# EUROPÄISCHE PATENTANMELDUNG

(11) **EP 4 764 579 A1**
(43) Veröffentlichungstag der Anmeldung: **24.06.2026**
(21) Anmeldenummer: 24220948.4
(22) Anmeldetag: 18.12.2024
(51) Int. Cl.: G01R 33/565, G01R 33/56, A61B 5/055, G01R 33/567

(54) **VERFAHREN ZUR ERSTELLUNG EINES OPTIMIERTEN MAGNETRESONANZ-BILDES**

(71) Anmelder: Siemens Healthineers AG, 91301 Forchheim (DE)
(72) Erfinder: Zeller, Mario, 91054 Erlangen (DE)
(74) Vertreter: Siemens Healthineers Patent Attorneys

(57) **Zusammenfassung**

Die vorliegende Erfindung betrifft ein computerimplementiertes Verfahren zur Erstellung eines optimierten Magnetresonanz-Bildes aufweisend:
Bereitstellen einer Vielzahl von Magnetresonanz-Echosignalen eines Magnetresonanz-Bildgebungsvorgangs eines Abbildungsgebietes (S10);
Bereitstellen einer Vielzahl von Navigatorsignalen, die zusammen mit den jeweiligen Magnetresonanz-Echosignalen erfasst wurden, und jeweils den jeweiligen Magnetresonanz-Echosignalen zugeordnet sind (S12);
Bestimmen eines Qualität-Werts des jeweiligen Navigatorsignals der Vielzahl der Navigatorsignale mittels eines trainierten maschinellen Navigatorsignal-Bewertungs-Lernmodells, insbesondere zur Bewertung des jeweiligen Navigatorsignals (S14);
Bestimmen einer Teilmenge der Vielzahl der Magnetresonanz-Echosignale, basierend auf den jeweiligen Qualitäts-Werten der Navigatorsignale, um insbesondere das Magnetresonanz-Bild zu verbessern (S16); und
Generieren des optimierten Magnetresonanz-Bildes mittels der Teilmenge der Vielzahl von Magnetresonanz-Echosignalen (S18).

## Beschreibung

Die vorliegende Erfindung betrifft ein computerimplementiertes Verfahren zur Erstellung eines optimierten Magnetresonanz-Bildes, ein computerimplementiertes Verfahren zur Bewertung eines Magnetresonanz-Echosignals, ein computerimplementiertes Verfahren zum Trainieren eines maschinellen Navigatorsignal-Bewertungs-Lernmodells und eine Magnetresonanzanlage, die eingerichtet ist, diese Verfahren auszuführen.

Magnetresonanztomographen bzw. Magnetresonanzanlagen können als bildgebende Vorrichtungen Abbildungen eines Abbildungsgebietes eines Untersuchungsobjektes generieren und dazu einen Teil der Kernspins des Untersuchungsobjektes mit einem starken äußeren Magnetfeld ausrichten und diese durch ein magnetisches Wechselfeld zur Präzession um diese Ausrichtung anregen. Die Präzession bzw. Rückkehr der Kernspins aus diesem angeregten Zustand in einen Zustand mit geringerer Energie wiederum erzeugt als Antwort ein magnetisches Wechselfeld, das über Antennen als Magnetresonanz-Echosignal empfangen werden kann.

Mit Hilfe von magnetischen Gradientenfeldern kann den Signalen eine Ortskodierung aufgeprägt werden, die nachfolgend eine Zuordnung von dem empfangenen Signal zu einem Volumenelement ermöglicht. Das empfangene Signal kann dann ausgewertet werden und eine zweidimensionale oder dreidimensionale bildgebende Darstellung des Untersuchungsobjektes als Magnetresonanz-Bild bereitstellen.

In diesem Zusammenhang hat sich herausgestellt, dass ein Bedarf besteht, dass Magnetresonanz-Bild, insbesondere in Bezug auf Bewegungsartefakte im Magnetresonanz-Bild, zu optimieren.

Es ist daher eine Aufgabe der vorliegenden Erfindung eine Lösung bereitzustellen, um ein Magnetresonanz-Bild basierend auf Magnetresonanz-Echosignal zu erstellen, welches in Bezug auf Bewegungsartefakte im Magnetresonanz-Bild optimiert ist.

Diese und andere Aufgaben, die beim Lesen der folgenden Beschreibung noch genannt werden oder vom Fachmann erkannt werden können, werden durch den Gegenstand der unabhängigen Ansprüche gelöst. Die abhängigen Ansprüche bilden den zentralen Gedanken der vorliegenden Erfindung in besonders vorteilhafter Weise weiter.

Ein erster Aspekt der vorliegenden Offenbarung betrifft ein computerimplementiertes Verfahren zur Erstellung eines optimierten Magnetresonanz-Bildes, welches die folgenden Schritte aufweist. In einem Schritt wird eine Vielzahl von Magnetresonanz-Echosignalen eines Magnetresonanz-Bildgebungsvorgangs eines Abbildungsgebietes bereitgestellt. In einem weiteren Schritt wird eine Vielzahl von Navigatorsignalen bereitgestellt, die zusammen mit den jeweiligen Magnetresonanz-Echosignalen des Magnetresonanz-Bildgebungsvorgangs des Abbildungsgebietes erfasst wurden, und jeweils den jeweiligen Magnetresonanz-Echosignalen zugeordnet, bzw. mit den jeweiligen Magnetresonanz-Echosignale und mit dem Magnetresonanz-Bildgebungsvorgang, assoziiert, sind. In einem weiteren Schritt wird ein Qualitäts-Wert des jeweiligen Navigatorsignals der Vielzahl der Navigatorsignale mittels eines trainierten maschinellen Navigatorsignal-Bewertungs-Lernmodells bestimmt, insbesondere um das jeweilige Navigatorsignal zu bewerten. In einem weiteren Schritt wird eine Teilmenge der Vielzahl der Magnetresonanz-Echosignale, basierend auf den jeweiligen Qualitäts-Werten der Navigatorsignale, bestimmt, um insbesondere das Magnetresonanz-Bild zu verbessern. D.h. mit anderen Worten, dass die Teilmenge der Vielzahl der Magnetresonanz-Echosignale abhängig von den jeweiligen Qualitäts-Werten der Navigatorsignale ausgesucht werden. Beispielsweise kann die Teilmenge der Vielzahl der Magnetresonanz-Echosignale dadurch bestimmt werden, dass Magnetresonanz-Echosignale deren zugeordnete Navigatorsignale einen Qualitäts-Wert unterhalb eines Grenzwertes aufweisen, verworfen werden. In einem weiteren Schritt wird das optimierte Magnetresonanz-Bild mittels der Teilmenge der Vielzahl von Magnetresonanz-Echosignalen generiert, insbesondere um Magnetresonanz-Bilder zu generieren, die in Bezug auf Bewegungsartefakte optimiert sind.

Ein Magnetresonanz-Echosignal eines Magnetresonanz-Bildgebungsvorgangs kann ein Signal zur Erstellung eines Magnetresonanz-Bildes sein, welches einen einzelnen Teilabschnitt eines k-Raums oder eine Mehrzahl von Teilabschnitten eines k-Raums für das Magnetresonanz-Bild definiert. Beispielsweise kann ein Magnetresonanz-Echosignal einen Echo-Zug oder einen Teil eines Echo-Zugs umfassen. Unter einem Echo-Zug wird insbesondere eine Serie von insbesondere rephasierenden Hochfrequenzpulsen und ihre entsprechenden Magnetresonanz-Echos verstanden.

Ein Navigatorsignal kann ein Signal sein, welches eingerichtet ist, räumliche und/oder zeitliche Veränderungen des Abbildungsgebietes zu charakterisieren. Das Navigatorsignal kann vor, nach und/oder während eines Generierens eines Magnetresonanz-Echosignals detektiert werden. Das Navigatorsignal ist insbesondere eine Zeitreihe von Messwerten, die während des Magnetresonanz-Bildgebungsvorgangs aufgenommen werden und den Bewegungszustand des Abbildungsgebietes, insbesondere des Patienten bzw. der abgebildeten Struktur im Abbildungsgebietes, charakterisieren. Ein Messwert enthält Informationen über die Position der abgebildeten Struktur im Abbildungsgebiet. Insbesondere enthält das Navigatorsignal einen oder mehrere Messwerte für jedes Magnetresonanz-Echosignal.

Vorteilhafterweise können bei diesem Verfahren zur Erstellung eines optimierten Magnetresonanz-Bildes Qualitäts-Werte von Navigatorsignale verwendet werden, um eine geeignete Teilmenge von Magnetresonanz-Echosignale zu bestimmen, mit der ein optimiertes Magnetresonanz-Bild erstellt werden kann. Dadurch können Magnetresonanz-Echosignale identifiziert werden, die beispielsweise aufgrund von Bewegungen des Abbildungsgebietes, Artefakte in einem Magnetresonanz-Bild hervorrufen könnten.

Dazu kann das maschinellen Navigatorsignal-Bewertungs-Lernmodell trainiert werden, um jedem einer Vielzahl von Navigatorsignalen einen Qualität-Wert zuzuordnen. Insbesondere kann dadurch analysiert werden, wie sehr ein Verwerfen eines Magnetresonanz-Echosignals die Qualität des Magnetresonanz-Bildes verbessern kann, beispielsweise in dem Bewegungsartefakte reduziert werden.

Vorteilhafterweise kann die Teilmenge der Vielzahl der Magnetresonanz-Echosignale mittels Verwerfens zumindest eines Magnetresonanz-Echosignals basierend auf dem jeweiligen Qualitäts-Wert aus der Vielzahl der Magnetresonanz-Echosignale bestimmt werden, wobei insbesondere eine maximale Anzahl von verworfenen Magnetresonanz-Echosignalen begrenzt ist.

Die maximale Anzahl von verworfenen Magnetresonanz-Echosignale kann beispielsweise auf 25 % der aufgenommen Magnetresonanz-Echosignale begrenzt werden, um eine ausreichende Qualität des Magnetresonanz-Bildes sicherzustellen.

Insbesondere kann eine Anzahl verworfener Magnetresonanz-Echosignale, die in einem k-Raum benachbart angeordnet sind, begrenzt sein. Besonders vorteilhafterweise kann nicht eine höhere Anzahl in dem k-Raum benachbart angeordnete Magnetresonanz-Echosignale verworfen werden als einer Verdoppelung, oder insbesondere einer Vervielfachung, vorteilhafterweise einer Vervielfachung um einen Faktor zwischen 2 und 8, einer lokalen Unterabtastung der Magnetresonanz-Echosignale in dem k-Raum entspricht. Dabei bezieht sich die lokale Unterabtastung auf eine Anordnung der Magnetresonanz-Echosignale in dem k-Raum.

Vorteilhafterweise erweitert das eine mögliche Auswahl der Magnetresonanz-Echosignale. Ebenso kann bei einer unregelmäßigen Unterabtastung der Ursprungsaufnahme, z.B. Compressed Sensing, eine lokal höhere Unterabtastung möglich sein.

Alternativ oder zusätzlich, kann zumindest ein Magnetresonanz-Echosignal mit einem Qualitäts-Wert, der kleiner als ein Limit-Wert ist, verworfen wird.

Das Navigatorsignal kann mittels eines Kamerasignals und/oder eines Videosignals und/oder eines Radarsignals und/oder eines RF-Reflexions-Signals und/oder eines Pilot-Tone-Signals generiert werden. Alternativ oder zusätzlich können Navigatorsignale generiert werden, welche auf einem Magnetresonanz-Signal basieren. Dazu werden zusätzliche RF-Impulse geschaltet, die zur dynamischen Verfolgung anatomischer Bewegungen verwendet werden.

Gemäß einem Aspekt, wird ein computerimplementiertes Verfahren zur Bewertung eines Magnetresonanz-Echosignals vorgeschlagen, wobei das Magnetresonanz-Echosignal insbesondere in Bezug auf eine Qualität eines Magnetresonanz-Bildes bewertet wird.

In einem Schritt des Verfahrens kann eine Vielzahl von Magnetresonanz-Echosignalen eines Magnetresonanz-Bildgebungsvorgangs eines Abbildungsgebietes bereitgestellt werden. In einem weiteren Schritt kann eine Vielzahl von Navigatorsignalen, die jeweils mit den Magnetresonanz-Echosignalen der Vielzahl der Magnetresonanz-Echosignale assoziiert sind, bereitgestellt werden. In einem weiteren Schritt des Verfahrens kann eine Vielzahl von ersten Teilmengen der Vielzahl der Magnetresonanz-Echosignale erstellt werden, wobei aus jeder ersten Teilmenge zumindest ein Magnetresonanz-Echosignal der Vielzahl der Magnetresonanz-Echosignale verworfen, bzw. entfernt worden, ist. Dabei kann jede Teilmenge der Vielzahl der ersten Teilmengen der Magnetresonanz-Echosignale so erstellt werden, dass die ersten Teilmengen in Bezug auf die enthaltenen Magnetresonanz-Echosignale unterschiedlich sind. In einem weiteren Schritt kann eine Vielzahl von ersten Magnetresonanz-Bildern rekonstruiert werden, wobei jedes erste Magnetresonanz-Bild der Vielzahl der ersten Magnetresonanz-Bilder auf einer anderen ersten Teilmenge der Vielzahl der Magnetresonanz-Echosignale basieren kann. In einem weiteren Schritt kann ein Qualitäts-Wert jedes ersten Magnetresonanz-Bildes der Vielzahl der ersten Magnetresonanz-Bilder, insbesondere mittels eines Bildqualität-Bewertungs-Netzwerks, bestimmt werden. In einem weiteren Schritt kann der Qualitäts-Wert des jeweiligen ersten Magnetresonanz-Bildes zu der jeweiligen ersten Teilmenge der assoziierten Navigatorsignale zugewiesen werden, um insbesondere bewertete Navigatorsignale in einem weiteren Schritt bereitstellen zu können.

Vorteilhafterweise können mit diesem Verfahren zur Bewertung von Magnetresonanz-Echosignalen Navigatorsignale, insbesondere mit einem Qualitäts-Wert, gelabelt bzw. bewertet werden, ohne dass manuelle Labelling-Schritte notwendig sind. Mit anderen Worten, können mit diesem Verfahren zur Bewertung von Magnetresonanz-Echosignalen Trainingsdatensätze für ein maschinelles Navigatorsignal-Bewertungs-Lernmodell mittels bestehender Magnetresonanz-Bildern, denen Navigatorsignale zugeordnet werden können, automatisch generiert werden, um insbesondere das maschinelle Navigatorsignal-Bewertungs-Lernmodell mit diesen Trainingsdatensätzen zu trainieren. Eine Auswahl von Magnetresonanz-Echosignale für das Generieren eines Magnetresonanz-Bildes kann ein Magnetresonanz-Bild optimieren, bzw. die Bildqualität steigern.

Bildqualität-Bewertungs-Netzwerke zur Bewertung von Magnetresonanz-Bildern sind bekannt und gehören zum Stand der Technik. Siehe beispielsweise: Automatic MR image quality evaluation using a Deep CNN: A reference-free method to rate motion artifacts in neuroimaging, Irene Fantini, et al, Computerized Medical Imaging and Graphics 90 (2021) 101897

Zusätzlich kann dem jeweiligen assoziierten Navigationssignal, dessen erstes Magnetresonanz-Echosignal in der jeweiligen Teilmenge der Vielzahl der ersten Teilmengen der Magnetresonanz-Echosignale verworfen wurde, um insbesondere ein verbessertes Magnetresonanz-Bild zu erhalten, ein um den Qualitäts-Wert des jeweiligen ersten Magnetresonanz-Bildes verminderten maximalen Qualität-Wert (d.h. z.B. 1-Q) zugewiesen werden, um insbesondere weitere bewertete Navigationssignale bereitzustellen. Mittels dieser Werte können einem Navigatorsignale-Bewertung-Lernmodell schlecht bewertete Navigationssignale als Trainingsdaten bereitgestellt werden.

Besonders vorzugsweise können die bewerteten Navigationssignale für ein Training eines Navigatorsignal-Bewertungs-Lernmodells bereitgestellt werden, wenn der Qualitäts-Wert zumindest eines ersten Magnetresonanz-Bildes größer ist als ein Qualitäts-Wert eines originären Magnetresonanz-Bildes, welches mittels der Vielzahl der bereitgestellten Magnetresonanz-Echosignale des Bildgebungsvorgangs rekonstruiert ist.

Mit anderen Worten kann mit dem Verfahren zur Bewertung eines Magnetresonanz-Echosignale identifiziert werden, welche Magnetresonanz-Echosignale verworfen werden können, um eine Qualität eines Magnetresonanz-Bildes zu verbessern. Dazu kann eine Vielzahl von Magnetresonanz-Echosignal-Kandidaten durch Kombinationen von beibehaltenen bzw. verworfenen Magnetresonanz-Echosignale und Rekonstruktion der Magnetresonanz-Bilder bestimmt werden. Dies kann beispielsweise für N=8 Magnetresonanz-Echosignale folgendermaßen in Vektoren dargestellt werden, wenn R für ein verworfenes Magnetresonanz-Echosignale steht und K für beibehalten des Magnetresonanz-Echosignale:
(K, K, K, K, K, K, K, K) (Referenz mit der Vielzahl von Magnetresonanz-Echosignale)
(R, K, K, K, K, K, K, K) (Teilmenge der Magnetresonanz-Echosignale)
(R, R, K, K, K, K, K, K)
(K, R, K, K, K, K, K, K)

Basierend auf einem existierenden und trainierten neuronalen Netz zur Beurteilung der Qualität eines Magnetresonanz-Bildes hinsichtlich Bewegung oder Einfaltungsartefakte, kann für die jeweilige Teilmenge verglichen werden, ob das Verwerfen eines Magnetresonanz-Echosignals die Bildqualität verbessert. Die dabei erhaltenen Qualitäts-Werte können den Navigatorsignalen, die zugehörig oder assoziiert zu dem jeweiligen Magnetresonanz-Echosignale bestimmt wurden, zugeordnet werden. Beibehaltene Magnetresonanz-Echosignale können beispielsweise mit einem Qualität-Wert zwischen null und eins bewertet oder gelabelt werden und die jeweiligen verworfenen Magnetresonanz-Echosignale können mit 1 - Q gelabelt werden. Ein Training eines Navigator-Klassifikationsnetzwerks, wie dem Navigatorsignal-Bewertungs-Lernmodell, mit diesen Labeln kann dazu führen, dass das Netzwerk darauf trainiert wird, die Ähnlichkeit als ein direktes Maß zu sehen, wie ein Verwerfen von n Echozügen die Bildqualität verbessert. Insbesondere kann zunächst ein Verwerfen von nur jeweils einem Magnetresonanz-Echosignale an jeder Position und einer Bewertung durch das Bewertungsnetzwerk hinsichtlich der Bildqualität erfolgen. Nur für die verworfenen Magnetresonanz-Echosignale, für die sich die Bildqualität des Magnetresonanz-Bildes gegenüber einem Referenz Magnetresonanz-Bild mit der Vielzahl der Magnetresonanz-Bilder verbessert, kann das Verfahren mit einem Verwerfen zusätzlicher Magnetresonanz-Bilder fortgesetzt werden. Besonders vorteilhafterweise kann dieses Verfahren so lange fortgesetzt werden, wie ein Verwerfen zusätzlicher Magnetresonanz-Echosignale zu einer weiteren Verbesserung der Bildqualität führt. Durch dieses Vorgehen wird die Zeit zum Finden von im Hinblick auf Bildqualitätsverbesserungen relevanten Magnetresonanz-Echosignal-Kandidaten verkürzt, da nicht mehr alle 2^N möglichen Kombinationen im Vektor untersucht werden müssen.

Weitere Trainingsdaten können generiert werden, wenn Navigatorsignale aus dem Abbildungsgebietes benachbarten Abbildungsgebieten auch mit dem Qualitäts-Wert des Magnetresonanz-Bildes des Abbildungsgebietes bewertet werden. Dadurch kann auch die Qualität des optimierten Magnetresonanz-Bildes verbessert werden. Wenn solche Nachbarschichten, bzw. benachbarte Abbildungsgebiete, in die Bewertung einbezogen werden, kann eine Vervielfachung der Trainingsdaten dadurch erfolgen, indem die Kandidaten der Einzelschichten beliebig miteinander kombiniert werden. Wenn eine Bildqualität durch das Weglassen eines Magnetresonanz-Echosignals nicht steigt, kann geschlossen werden, dass der Magnetresonanz-Bildgebungsvorgang nicht von einer störenden Bewegung betroffen ist.

Vorteilhafterweise ermöglicht das beschriebene Verfahren zur Bewertung eines Magnetresonanz-Echosignals, aus einem von Bewegung betroffenen Datensatz eine Vielzahl von automatisch gelabelten Trainingsdatensätzen zu erzeugen. Damit entfällt das gezielte Aufnehmen von Daten ohne jeglichen Bewegungseinfluss bzw. das Aufprägen von künstlicher Bewegung auf bewegungsfreie Daten. Andererseits können künstlich erzeugte Datensätze (Navigator + k-Raumdaten) automatisch annotiert werden.

Vorteilhafterweise kann das computerimplementierte Verfahren zur Bewertung eines Magnetresonanz-Echosignals einen um den Qualitäts-Wert des jeweiligen ersten Magnetresonanz-Bildes verminderten maximalen Qualitäts-Wert zu dem jeweiligen assoziierten Navigatorsignal des jeweiligen zumindest einen verworfenen Magnetresonanz-Echosignal zuweisen, um insbesondere bewertete Navigatorsignale bereitzustellen. D.h. mit anderen Worten, dass dem verworfenen Magnetresonanz-Echosignale beispielsweise ein Wert 1 - Q zugeordnet werden kann, um in den Trainingsdaten für das Navigatorsignal-Bewertungs-Lernmodell schlecht bewertete Magnetresonanz-Echosignale bereitzustellen.

Entsprechend einem Aspekt kann die Vielzahl von Magnetresonanz-Echosignalen Magnetresonanz-Echosignale eines Magnetresonanz-Bildgebungsvorgangs zumindest eines dem Abbildungsgebiet benachbarten Abbildungsgebiet umfassen. Alternativ oder zusätzlich, kann die Vielzahl von Magnetresonanz-Echosignalen eine Vielzahl von Magnetresonanz-Echosignale einer Vielzahl von zeitlich aufeinanderfolgenden Magnetresonanz-Bildgebungsvorgängen des Abbildungsgebiets umfassen.

Insbesondere können diese Magnetresonanz-Echosignale die zu unterschiedlichen Zeitpunkten aufgenommen wurden und/oder aus unterschiedlichen Abbildungsgebieten generiert wurden, für ein Training des Navigatorsignal-Bewertungs-Lernmodell in einer zugehörigen Eingangsschicht des Lernmodells zeitlich und/oder räumlich angeordnet werden.

Beispielsweise können unterschiedliche Schichten oder Abbildungsgebietes in drei sog. "interleaves" aufgenommen werden. In einem ersten Zeitabschnitt sind dann die Schichten 1, 4, 7, in einem zweiten Zeitabschnitt die Schichten 2, 5, 8, in einem dritten Zeitabschnitt die Schichten 3, 6, 9, die für die Trainingsdaten vorteilhafterweise entsprechend räumlich und/oder zeitlich sortiert werden können.

Eine räumliche und zeitliche Anordnung der Navigatorsignale kann beispielsweise mittels "postional encoding" erreicht werden.

Also kann insbesondere die Vielzahl von Magnetresonanz-Echosignale der Vielzahl von zeitlich aufeinanderfolgenden Magnetresonanz-Bildgebungsvorgängen des Abbildungsgebiets entsprechend der zeitlichen Abfolge in der Eingangsschicht angeordnet sein. Zusätzlich oder alternativ, können die Vielzahl von Magnetresonanz-Echosignale aus dem zumindest den Abbildungsgebiet benachbarten Abbildungsgebiet in der Eingangsschicht entsprechend der räumlichen Anordnung angeordnet sein.

Vorteilhafterweise kann das computerimplementierte Verfahren zur Bewertung eines Magnetresonanz-Echosignals die folgenden weiteren Schritte aufweisen. In einem Schritt kann ein originäres Magnetresonanz-Bild mittels der Vielzahl der bereitgestellten Magnetresonanz-Echosignale des Bildgebungsvorgangs des Abbildungsgebietes rekonstruiert werden. In einem weiteren Schritt kann ein Qualitäts-Wert des originären Magnetresonanz-Bildes, insbesondere mittels des Bildqualität-Bewertungs-Netzwerks, bestimmt werden. In einem weiteren Schritt kann der Qualitäts-Wert jedes der ersten Magnetresonanz-Bilder mit dem Qualitäts-Wert des originären Magnetresonanz-Bildes verglichen werden. In einem weiteren Schritt kann, basierend auf einer Höhe des jeweiligen Qualitäts-Wertes entschieden werden, ob weitere Schritte des Verfahrens durchgeführt werden. Wenn der Vergleich das originären Magnetresonanz-Bild präferiert, beispielsweise wenn der Qualitäts-Wert zumindest eines ersten Magnetresonanz-Bildes größer ist als der Qualitäts-Wert des originären Magnetresonanz-Bildes, können die folgenden weiteren Schritte durchgeführt werden.

In einem weiteren Schritt kann eine Vielzahl von zweiten Teilmengen der Vielzahl der Magnetresonanz-Echosignale, mittels Verwerfen zumindest eines weiteren Magnetresonanz-Echosignals aus jeder ersten Teilmenge der Vielzahl der Magnetresonanz-Echosignale erstellt werden, wobei jede zweite Teilmenge der Vielzahl der Magnetresonanz-Echosignale so erstellt wird, dass jede zweite Teilmenge in Bezug auf die enthaltenen Magnetresonanz-Echosignale unterschiedlich ist. In einem weiteren Schritt kann eine Vielzahl von zweiten Magnetresonanz-Bildern rekonstruiert werden, wobei jedes zweite Magnetresonanz-Bild der Vielzahl der zweiten Magnetresonanz-Bilder auf einer anderen zweiten Teilmenge der Vielzahl der Magnetresonanz-Echosignale basieren kann. In einem weiteren Schritt kann ein Qualitäts-Wert jedes der Vielzahl der zweiten Magnetresonanz-Bilder, insbesondere mittels des Bildqualität-Bewertungs-Netzwerks, bestimmt werden. In einem weiteren Schritt kann der Qualitäts-Wert des jeweiligen zweiten Magnetresonanz-Bildes der Vielzahl der zweiten Magnetresonanz-Bilder zu einer jeweiligen zweiten Teilmenge der assoziierten Navigatorsignale zugewiesen werden, die zu der zweiten Teilmenge der Vielzahl der Magnetresonanz-Echosignale zugeordnet, bzw. assoziiert, sind, um insbesondere in einem Schritt eine Vielzahl von weiteren bewerteten Navigatorsignalen bereitzustellen zu können.

Vorteilhafterweise werden bei diesem Verfahren bewertete Navigationssignale dann generiert, wenn eine Bewertung zumindest eines der ersten Magnetresonanz-Bilder besser ist als die Bewertung des originären Magnetresonanz-Bildes.

Zusätzlich kann dem jeweiligen assoziierten Navigationssignal, dessen zweites Magnetresonanz-Echosignal in der jeweiligen Teilmenge der Vielzahl der zweiten Teilmengen der Magnetresonanz-Echosignale verworfen wurde, um insbesondere ein verbessertes Magnetresonanz-Bild zu erhalten, ein um den Qualitäts-Wert des jeweiligen zweiten Magnetresonanz-Bildes verminderten maximalen Qualität-Wert (d.h. z.B. 1-Q) zugewiesen werden, um insbesondere weitere bewertete Navigationssignale bereitzustellen. Mittels dieser Werte können einem Navigatorsignale-Bewertung-Lernmodell schlecht bewertete Navigationssignale als Trainingsdaten bereitgestellt werden.

Das computerimplementierte Verfahren zur Bewertung eines Magnetresonanz-Echosignals kann vorteilhafterweise die folgenden weiteren Schritte aufweisen.

In einem Schritt des Verfahrens kann ein Referenz-Magnetresonanz-Bild aus der Vielzahl der zweiten Magnetresonanz-Bilder, basierend auf einer Höhe des jeweiligen Qualitäts-Wertes, ausgewählt werden. In einem weiteren Schritt kann das Referenz-Magnetresonanz-Bild mit zumindest einer Teilmenge der ersten Magnetresonanz-Bilder, basierend auf einer Höhe des jeweiligen Qualitäts-Wertes, verglichen werden. In einem weiteren Schritt können, wenn der Vergleich das Referenz-Magnetresonanz-Bildes präferiert, die folgenden Schritte ausgeführt werden.

In einem weiteren Schritt des Verfahrens kann eine Vielzahl von dritten Teilmengen der Vielzahl der Magnetresonanz-Echosignale, mittels Verwerfen zumindest eines weiteren Magnetresonanz-Echosignals aus jeder zweiten Teilmenge der Vielzahl der Magnetresonanz-Echosignale erstellt werden, wobei jede dritte Teilmenge der Vielzahl der Magnetresonanz-Echosignale in Bezug auf die enthaltenen Magnetresonanz-Echosignale unterschiedlich ist. In einem weiteren Schritt kann eine Vielzahl von dritten Magnetresonanz-Bildern rekonstruiert werden, wobei jedes dritte Magnetresonanz-Bild der Vielzahl der dritten Magnetresonanz-Bilder auf einer anderen dritten Teilmenge der Vielzahl der Magnetresonanz-Echosignale basiert. In einem weiteren Schritt kann ein Qualitäts-Wert jedes der Vielzahl der dritten Magnetresonanz-Bilder, insbesondere mittels des Bildqualität-Bewertungs-Netzwerks, bestimmt werden. In einem weiteren Schritt können die Bewertung des jeweiligen dritten Magnetresonanz-Bildes der Vielzahl der dritten Magnetresonanz-Bilder zu jeweiligen dritten Teilmengen der assoziierten Navigatorsignale zugewiesen werden, um in einem weiteren Schritt insbesondere eine Vielzahl von bewerteten Navigatorsignalen bereitzustellen.

Basierend auf dem jeweiligen Qualitäts-Wert kann ein Präferieren eines Magnetresonanz-Bildes gegenüber einem anderen Magnetresonanz-Bild mit komplexen Algorithmen erfolgen. Insbesondere kann das Magnetresonanz-Bild präferiert werden, welches einen höheren Qualitäts-Wert aufweist.

Zusätzlich zu den oben beschriebenen Schritten, kann dem jeweiligen assoziierten Navigationssignal, dessen drittes Magnetresonanz-Echosignal in der jeweiligen Teilmenge der Vielzahl der dritten Teilmengen der Magnetresonanz-Echosignale verworfen wurde, um insbesondere ein verbessertes Magnetresonanz-Bild zu erhalten, ein um den Qualitäts-Wert des jeweiligen dritten Magnetresonanz-Bildes verminderten maximalen Qualitäts-Wert (d.h. z.B. 1-Q) zugewiesen werden, um insbesondere weitere bewertete Navigationssignale bereitzustellen. Mittels dieser Werte können einem Navigatorsignale-Bewertung-Lernmodell schlecht bewertete Navigationssignale als Trainingsdaten bereitgestellt werden.

Gemäß einem Aspekt, wird ein computerimplementiertes Verfahren zum Training eines maschinellen Navigatorsignal-Bewertung-Lernmodells vorgeschlagen, wobei das trainierte Navigatorsignal-Bewertung-Lernmodell eingerichtet ist, Navigatorsignale beispielsweise mit einem Qualitäts-Wert, zu bewerten. Das Training des maschinellen Navigatorsignal-Bewertung-Lernmodells kann beispielsweise mit einem überwachten Lernen erfolgen. In einem Schritt des Verfahrens wird eine Vielzahl von Teilmengen von Navigatorsignalen bereitgestellt. In einem weiteren Schritt wird eine Vielzahl von Bewertungen zu den jeweiligen Teilmengen der Navigatorsignale bereitgestellt. In einem weiteren Schritt, bzw. in einer Abfolge von weiteren Schritten, wird das maschinelle Navigatorsignal-Bewertungs-Lernmodell mittels der Vielzahl der Teilmengen der Navigatorsignale als Eingangsgröße und der jeweils zugeordneten Qualitäts-Werte als Zielgröße, bzw. Ausgangsgröße, adaptiert, um eine Abweichung der Bewertung des jeweiligen Navigatorsignals von dem jeweiligen Qualitäts-Wert zu minimieren. Das Adaptieren des maschinellen Navigatorsignale-Bewertung-Lernmodells kann auf der Grundlage eines Backpropagation-Algorithmus erfolgen. Um eine Überanpassung zu verhindern, können Regularisierungsmethoden verwendet werden. Insbesondere kann das trainierte maschinellen Navigatorsignale-Bewegung-Lernmodell zum Bestimmen von Qualität-Werten von Navigatorsignale bereitgestellt werden.

Eine Architektur des maschinellen Navigatorsignal-Bewertung-Lernmodells kann als neuronales Faltungs-Netzwerk zur Bewertung von Navigatorsignalen ausgebildet sein und eingerichtet sein, Navigatorsignale, beispielsweise mit einem Qualität-Wert, zu bewerten. D.h. die Eingangssignale kann eine Vielfalt von Navigatorsignalen eines Magnetresonanz-Bildgebungsvorgangs eines Abbildungsgebiets sein, wobei insbesondere der Abbildungsvorgang mittels einer Magnetresonanzanlage durchgeführt werden kann.

Die Vielfalt der Navigatorsignale kann diesem neuronalen Faltungs-Netzwerk mittels einer Eingangsknoten-Schicht bereitgestellt werden.

Die Eingangsknoten-Schicht kann eine variable Größe aufweisen und insbesondere für eine Anzahl von N Navigatoren der Länge 128 Pixel ausgestaltet sein. Insbesondere kann das neuronale Faltung-Netzwerk eingerichtet sein eine Mehrzahl M von Schichten, bzw. Abbildungsgebieten gemeinsam zu betrachten. In diesem Fall kann die Vielfalt der Navigatorsignale eine Anordnung in der Form (N, M,128) oder (N*M, 128) aufweisen. Die Vielfalt der Navigatorsignale kann in der Eingangsknoten-Schicht entsprechend ihrer räumlichen Anordnung und/oder ihrer zeitlichen Abfolge angeordnet sein, um räumliche und/oder zeitliche Merkmale, bzw. Korrelationen, mittels des neuronalen Faltung-Netzwerks zu extrahieren. Beispielsweise kann so eine kurze Bewegung innerhalb von Echozügen von 8 von 20 Schichten in einer Repetitions-Periode TR effektiv genutzt werden.

Eine erste und eine zweite Faltungsschicht mit jeweils einer nachfolgenden Pooling-Schicht kann eingerichtet sein, Merkmale der Vielfalt der Navigatorsignale zu extrahieren. Eine Flatten-Schicht kann eingerichtet sein, Ausgangssignale des neuronalen Faltungs-Netzwerks eindimensional darzustellen und mit einer nachfolgenden vollständig verbundenen Schicht zusammenzuwirken um Qualität-Werte den jeweiligen Navigatorsignalen an einer Ausgangsknoten-Schicht in einem Wertebereich zwischen null und eins bereitzustellen.

In einer vorteilhaften Ausführungsform kann zwischen zwei Schichten des neuronalen Netzwerkes eine konventionelle Ähnlichkeitsberechnung, beispielsweise eine Kosinus-Ähnlichkeitsberechnung, durchgeführt werden. Dies bietet den Vorteil, dass ein so gebildetes neuronales Netzwerk der Problemstruktur des Findens von ähnlichen Navigatorsignalen entsprechend, stabilisiert werden kann.

Gemäß einem Aspekt wird ein computerimplementiertes Verfahren zur Erstellung eines optimierten Magnetresonanz-Bildes vorgeschlagen, bei dem ein optimiertes Magnetresonanz-Bild mit einem der oben beschriebenen computerimplementierten Verfahren zur Erstellung eines optimierten Magnetresonanz-Bildes erstellt wird.

Dabei kann das maschinelle Navigatorsignal-Bewertungs-Lernmodell, gemäß einem oben beschriebenen computerimplementierten Verfahren zum Trainieren eines maschinellen Navigatorsignal-Bewertungs-Lernmodells zur Bewertung eines Navigatorsignals mit einem Qualitäts-Wert, mit bewerteten Navigatorsignalen trainiert werden.

Weiterhin können die Navigatorsignale, gemäß einem der oben beschriebene computerimplementierten Verfahren zur Bewertung eines Magnetresonanz-Echosignals, bewertet werden, um die bewertete Navigatorsignale bereitzustellen.

In einem Beispiel können bewertete Navigatorsignale bereitgestellt werden, indem ein Qualitäts-Wert Q eines Magnetresonanz-Bildes zu einer jeweiligen Teilmenge der assoziierten Navigatorsignale zugeordnet werden. Alternativ oder zusätzlich, können bewertete Navigatorsignale bereitgestellt werden, indem zu dem jeweiligen assoziierten Navigatorsignal des jeweiligen zumindest einen verworfenen Magnetresonanz-Echosignal ein um den Qualitäts-Wert des jeweiligen ersten Magnetresonanz-Bildes verminderten maximalen Qualitäts-Wert zugewiesen wird. Diese Zuweisung kann insbesondere dann erfolgen, wenn der Qualitäts-Wert zumindest eines mit zumindest einem verworfenen Magnetresonanz-Echosignal regenerierten Magnetresonanz-Bildes größer ist als ein Qualitäts-Wert eines originären Magnetresonanz-Bildes. Dadurch können bewertete Navigatorsignale bereitgestellt werden, die schlecht bewertet sind.

Insbesondere können mit diesen Verfahren Magnetresonanz-Bilder generiert werden, die in Bezug auf Bewegungsartefakte optimiert sind.

Gemäß einem Aspekt wird eine Magnetresonanzanlage mit einem Hauptmagneten, einem Gradientensystem, einem Hochfrequenzsystem und einer Steuereinheit vorgeschlagen. Die Steuereinheit kann mit dem Hauptmagneten, dem Gradientensystem und dem Hochfrequenzsystem gekoppelt sein, und dabei kann die Steuereinheit eingerichtet sein, eines der oben beschriebenen Verfahren mittels der Magnetresonanzanlage auszuführen.

Es wird ein Computerprogramm vorgeschlagen, welches direkt in einen Speicher einer Steuereinheit einer Magnetresonanzanlage ladbar ist, und Programm-Mittel aufweist, um die Schritte eines der oben beschriebenen Verfahren auszuführen, wenn das Programm in der Steuereinheit der Magnetresonanzanlage ausgeführt wird.

Es wird ein elektronisch lesbarer Datenträger mit darauf gespeicherten elektronisch lesbaren Steuerinformationen vorgeschlagen, welche zumindest ein oben beschriebenes Computerprogramm umfassen und derart ausgestaltet sind, dass sie bei Verwendung des Datenträgers in einer Steuereinheit einer Magnetresonanzanlage eines der oben beschriebenen Verfahren durchführen kann.

Es wird eine Verwendung eines Navigatorsignals vorgeschlagen, wobei das Navigatorsignal zusammen mit einem Magnetresonanz-Echosignal erfasst wurde, und das Navigatorsignal zur Bestimmung einer Teilmenge von Magnetresonanz-Echosignalen aus einer Vielzahl von Magnetresonanz-Echosignalen verwendet wird, um eine Rekonstruktion eines verbesserten Magnetresonanz-Bildes entsprechend einem der oben beschriebenen Verfahren durchzuführen.

Nachfolgend werden die beschriebenen Verfahren zur Erstellung eines optimierten Magnetresonanz-Bildes und zur Bewertung eines Magnetresonanz-Echosignals näher erläutert.

Alle hierin beschriebenen Ausführungsformen können miteinander kombiniert werden, soweit nicht explizit etwas anderes angegeben ist. Weitere Merkmale, Vorteile und

Anwendungsmöglichkeiten der vorliegenden Erfindung ergeben sich aus der nachfolgenden Beschreibung, dem Ausführungsbeispiel und den Figuren. Darin zeigen:
- Figur 1: skizziert in einem Flussdiagramm Schritte eines Verfahrens zur Erstellung eines optimierten Magnetresonanz-Bildes;
- Figur 2: skizziert in einem Flussdiagramm Schritte eines Verfahrens zur Bewertung von Magnetresonanz-Echosignalen;
- Figur 3: skizziert in einem Flussdiagramm Schritte zum Training eines maschinellen Navigatorsignal-Bewertung-Lernmodells;
- Figur 4: skizziert einen Aufbau eines Navigatorsignal-Bewertung-Lernmodells;
- Figur 5: Magnetresonanz-Bild;
- Figur 6: Magnetresonanz-Bild;
- Figur 7: Magnetresonanz-Bild; und
- Figur 8: skizziert eine Magnetresonanzanlage.

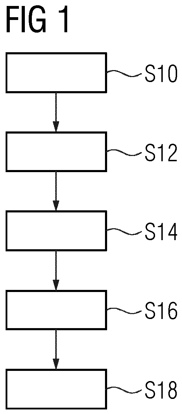

Die Figur 1 skizziert Schritte eines computerimplementierten Verfahrens zur Erstellung eines optimierten Magnetresonanz-Bildes welches die folgenden Schritte aufweist. In einem Schritt S10 wird eine Vielzahl von Magnetresonanz-Echosignalen eines Magnetresonanz-Bildgebungsvorgangs eines Abbildungsgebietes bereitgestellt. In einem weiteren Schritt S12 wird eine Vielzahl von Navigatorsignalen bereitgestellt, die zusammen mit den jeweiligen Magnetresonanz-Echosignalen des Magnetresonanz-Bildgebungsvorgangs des Abbildungsgebietes erfasst wurden, und jeweils den jeweiligen Magnetresonanz-Echosignalen zugeordnet, bzw. mit den jeweiligen Magnetresonanz-Echosignale und mit dem Magnetresonanz-Bildgebungsvorgang, assoziiert, sind. In einem weiteren Schritt S14 wird ein Qualitäts-Wert des jeweiligen Navigatorsignals der Vielzahl der Navigatorsignale mittels eines trainierten maschinellen Navigatorsignal-Bewertungs-Lernmodells bestimmt, insbesondere um das jeweilige Navigatorsignal zu bewerten. In einem weiteren Schritt S16 wird eine Teilmenge der Vielzahl der Magnetresonanz-Echosignale, basierend auf den jeweiligen Qualitäts-Werten der Navigatorsignale, bestimmt, um insbesondere das Magnetresonanz-Bild zu verbessern. D.h. mit anderen Worten, dass die Teilmenge der Vielzahl der Magnetresonanz-Echosignale abhängig von den jeweiligen Qualitäts-Werten der Navigatorsignale ausgesucht werden. Beispielsweise kann die Teilmenge der Vielzahl der Magnetresonanz-Echosignale dadurch bestimmt werden, dass Magnetresonanz-Echosignale deren zugeordnete Navigatorsignale einen Qualitäts-Wert unterhalb eines Grenzwertes aufweisen, verworfen werden. In einem weiteren Schritt S18 wird das optimierte Magnetresonanz-Bild mittels der Teilmenge der Vielzahl von Magnetresonanz-Echosignalen generiert, insbesondere um Magnetresonanz-Bilder zu generieren, die in Bezug auf Bewegungsartefakte optimiert sind.

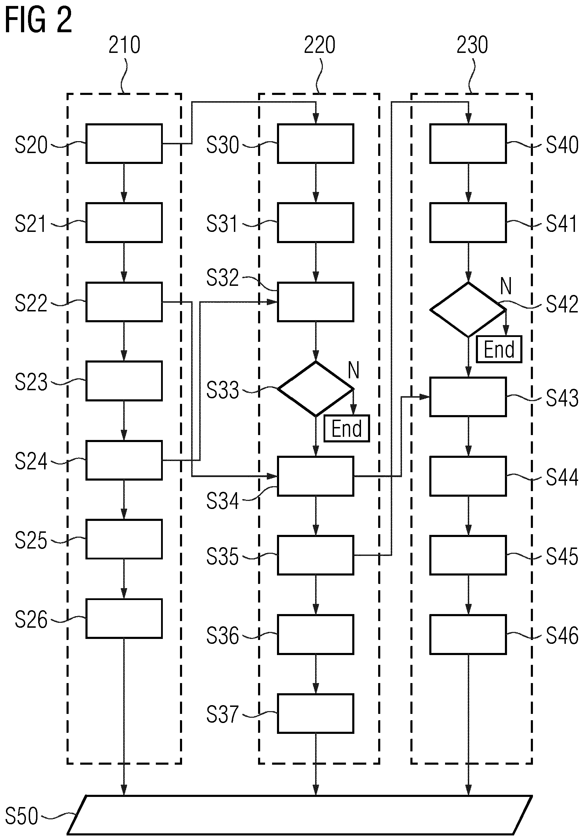

Die Figur 2 skizziert in einer ersten Sequenz 210 eines Flussdiagramms Schritte eines computerimplementierten Verfahrens zur Bewertung eines Magnetresonanz-Echosignals, wobei das Magnetresonanz-Echosignal insbesondere in Bezug auf eine Qualität eines Magnetresonanz-Bildes bewertet wird.

In einem Schritt S20 wird eine Vielzahl von Magnetresonanz-Echosignalen eines Magnetresonanz-Bildgebungsvorgangs eines Abbildungsgebietes bereitgestellt. In einem weiteren Schritt S21 wird eine Vielzahl von Navigatorsignalen, die jeweils mit den Magnetresonanz-Echosignalen der Vielzahl der Magnetresonanz-Echosignale assoziiert sind, bereitgestellt. In einem weiteren Schritt S22 wird eine Vielzahl von ersten Teilmengen der Vielzahl der Magnetresonanz-Echosignale erstellt, wobei aus jeder ersten Teilmenge zumindest ein Magnetresonanz-Echosignal der Vielzahl der Magnetresonanz-Echosignale verworfen, bzw. entfernt worden, ist. Dabei wird jede Teilmenge der Vielzahl der ersten Teilmengen der Magnetresonanz-Echosignale so erstellt, dass die ersten Teilmengen in Bezug auf die enthaltenen Magnetresonanz-Echosignale unterschiedlich sind. In einem weiteren Schritt S23 wird eine Vielzahl von ersten Magnetresonanz-Bildern rekonstruiert, wobei jedes erste Magnetresonanz-Bild der Vielzahl der ersten Magnetresonanz-Bilder auf einer anderen ersten Teilmenge der Vielzahl der Magnetresonanz-Echosignale basiert. In einem weiteren Schritt S24 wird ein Qualitäts-Wert jedes ersten Magnetresonanz-Bildes der Vielzahl der ersten Magnetresonanz-Bilder, insbesondere mittels eines Bildqualität-Bewertungs-Netzwerks, bestimmt. In einem weiteren Schritt S26 wird der Qualitäts-Wert des jeweiligen ersten Magnetresonanz-Bildes zu der jeweiligen ersten Teilmenge der assoziierten Navigatorsignale zugewiesen, um insbesondere bewertete Navigatorsignale im Schritt S50 bereitzustellen.

Zusätzlich kann dem jeweiligen assoziierten Navigationssignal, dessen erstes Magnetresonanz-Echosignal in der jeweiligen Teilmenge der Vielzahl der ersten Teilmengen der Magnetresonanz-Echosignale verworfen wurde, um insbesondere ein verbessertes Magnetresonanz-Bild zu erhalten, ein um den Qualitäts-Wert des jeweiligen ersten Magnetresonanz-Bildes verminderten maximalen Qualität-Wert (d.h. z.B. 1-Q) zugewiesen werden, um insbesondere weitere bewertete Navigationssignale bereitzustellen. Mittels dieser Werte können einem Navigatorsignale-Bewertung-Lernmodell schlecht bewertete Navigationssignale als Trainingsdaten bereitgestellt werden.

Besonders vorzugsweise können die bewerteten Navigationssignale für ein Training eines Navigatorsignal-Bewertungs-Lernmodells bereitgestellt werden, wenn der Qualitäts-Wert zumindest eines ersten Magnetresonanz-Bildes größer ist als ein Qualitäts-Wert eines originären Magnetresonanz-Bildes, welches mittels der Vielzahl der bereitgestellten Magnetresonanz-Echosignale des Bildgebungsvorgangs rekonstruiert ist.

Die Figur 2 skizziert in einer zweiten Sequenz 220 des Flussdiagramms weitere Schritte eines computerimplementierten Verfahrens zur Bewertung eines Magnetresonanz-Echosignals, wobei die zweite Sequenz 220 auch unabhängig von der ersten Sequenz 210 durchgeführt werden kann, wenn die Schritte S20, S22 und S24 der ersten Sequenz ergänzt werden.

In einem Schritt S30 der zweiten Sequenz wird ein originäres Magnetresonanz-Bild mittels der Vielzahl der bereitgestellten Magnetresonanz-Echosignale des Bildgebungsvorgangs des Abbildungsgebietes rekonstruiert. In einem weiteren Schritt S31 wird ein Qualitäts-Wert des originären Magnetresonanz-Bildes, insbesondere mittels des Bildqualität-Bewertungs-Netzwerks, bestimmt. In einem weiteren Schritt S32 wird der Qualitäts-Wert jedes der ersten Magnetresonanz-Bilder mit dem Qualitäts-Wert des originären Magnetresonanz-Bildes verglichen. In einem weiteren Schritt S33 wird, basierend auf einer Höhe des jeweiligen Qualitäts-Wertes entschieden, ob weitere Schritte des Verfahrens durchgeführt werden, und, wenn der Vergleich das originären Magnetresonanz-Bild präferiert, beispielsweise wenn der Qualitäts-Wert zumindest eines ersten Magnetresonanz-Bildes größer ist als der Qualitäts-Wert des originären Magnetresonanz-Bildes, werden die weiteren Schritte durchgeführt.

Wenn weitere Schritte des Verfahrens durchgeführt werden, wird im Schritt S34 eine Vielzahl von zweiten Teilmengen der Vielzahl der Magnetresonanz-Echosignale, mittels Verwerfen zumindest eines weiteren Magnetresonanz-Echosignals aus jeder ersten Teilmenge der Vielzahl der Magnetresonanz-Echosignale erstellt, wobei jede zweite Teilmenge der Vielzahl der Magnetresonanz-Echosignale so erstellt wird, dass jede zweite Teilmenge in Bezug auf die enthaltenen Magnetresonanz-Echosignale unterschiedlich ist. In einem weiteren Schritt S35 wird eine Vielzahl von zweiten Magnetresonanz-Bildern rekonstruiert, wobei jedes zweite Magnetresonanz-Bild der Vielzahl der zweiten Magnetresonanz-Bilder auf einer anderen zweiten Teilmenge der Vielzahl der Magnetresonanz-Echosignale basiert. In einem weiteren Schritt S36 wird ein Qualität-Wert jedes der Vielzahl der zweiten Magnetresonanz-Bilder, insbesondere mittels des Bildqualität-Bewertungs-Netzwerks, bestimmt. In einem weiteren Schritt S37 wird der Qualitäts-Wert des jeweiligen zweiten Magnetresonanz-Bildes der Vielzahl der zweiten Magnetresonanz-Bilder zu einer jeweiligen zweiten Teilmenge der assoziierten Navigatorsignale zugewiesen, die zu der zweiten Teilmenge der Vielzahl der Magnetresonanz-Echosignale zugeordnet, bzw. assoziiert, sind, um insbesondere in einem Schritt S50 eine Vielzahl von weiteren bewerteten Navigatorsignalen bereitzustellen.

Zusätzlich kann dem jeweiligen assoziierten Navigationssignal, dessen zweites Magnetresonanz-Echosignal in der jeweiligen Teilmenge der Vielzahl der zweiten Teilmengen der Magnetresonanz-Echosignale verworfen wurde, um insbesondere ein verbessertes Magnetresonanz-Bild zu erhalten, ein um den Qualitäts-Wert des jeweiligen zweiten Magnetresonanz-Bildes verminderten maximalen Qualität-Wert (d.h. z.B. 1-Q) zugewiesen werden, um insbesondere weitere bewertete Navigationssignale bereitzustellen. Mittels dieser Werte können einem Navigatorsignale-Bewertung-Lernmodell schlecht bewertete Navigationssignale als Trainingsdaten bereitgestellt werden.

Die Figur 2 skizziert in einer dritte Sequenz 230 des Flussdiagramms weitere Schritte eines computerimplementierten Verfahrens zur Bewertung eines Magnetresonanz-Echosignals, wobei die dritte Sequenz 230 auch unabhängig von der ersten Sequenz 210 und/oder der zweiten Sequenz 220 durchgeführt werden kann, wenn vorbereitende Verfahrens-Schritte der ersten Sequenz 210 und/oder der zweiten Sequenz 220 ergänzt werden.

In einem Schritt S40 der zweiten Sequenz wird ein Referenz-Magnetresonanz-Bild aus der Vielzahl der zweiten Magnetresonanz-Bilder, basierend auf einer Höhe des jeweiligen Qualitäts-Wertes, ausgewählt.

In einem weiteren Schritt S41 wird das Referenz-Magnetresonanz-Bild mit zumindest einer Teilmenge der ersten Magnetresonanz-Bilder, basierend auf einer Höhe des jeweiligen Qualitäts-Wertes, verglichen. In einem weiteren Schritt S42 werden, wenn der Vergleich das Referenz-Magnetresonanz-Bildes präferiert, die folgenden Schritte ausgeführt.

Im Schritt S43 wird eine Vielzahl von dritten Teilmengen der Vielzahl der Magnetresonanz-Echosignale, mittels Verwerfen zumindest eines weiteren Magnetresonanz-Echosignals aus jeder zweiten Teilmenge der Vielzahl der Magnetresonanz-Echosignale erstellt, wobei jede dritte Teilmenge der Vielzahl der Magnetresonanz-Echosignale in Bezug auf die enthaltenen Magnetresonanz-Echosignale unterschiedlich ist. In einem weiteren Schritt S44 wird eine Vielzahl von dritten Magnetresonanz-Bildern rekonstruiert, wobei jedes dritte Magnetresonanz-Bild der Vielzahl der dritten Magnetresonanz-Bilder auf einer anderen dritten Teilmenge der Vielzahl der Magnetresonanz-Echosignale basiert. In einem weiteren Schritt S45 wird ein Qualitäts-Wert jedes der Vielzahl der dritten Magnetresonanz-Bilder, insbesondere mittels des Bildqualität-Bewertungs-Netzwerks, bestimmt. In einem weiteren Schritt S46 wird die Bewertung des jeweiligen dritten Magnetresonanz-Bildes der Vielzahl der dritten Magnetresonanz-Bilder zu jeweiligen dritten Teilmengen der assoziierten Navigatorsignale zugewiesen, um im Schritt S 50 insbesondere eine Vielzahl von bewerteten Navigatorsignalen bereitzustellen.

Zusätzlich kann dem jeweiligen assoziierten Navigationssignal, dessen drittes Magnetresonanz-Echosignal in der jeweiligen Teilmenge der Vielzahl der dritten Teilmengen der Magnetresonanz-Echosignale verworfen wurde, um insbesondere ein verbessertes Magnetresonanz-Bild zu erhalten, ein um den Qualitäts-Wert des jeweiligen dritten Magnetresonanz-Bildes verminderten maximalen Qualitäts-Wert (d.h. z.B. 1-Q) zugewiesen werden, um insbesondere weitere bewertete Navigationssignale bereitzustellen. Mittels dieser Werte können einem Navigatorsignale-Bewertung-Lernmodell schlecht bewertete Navigationssignale als Trainingsdaten bereitgestellt werden.

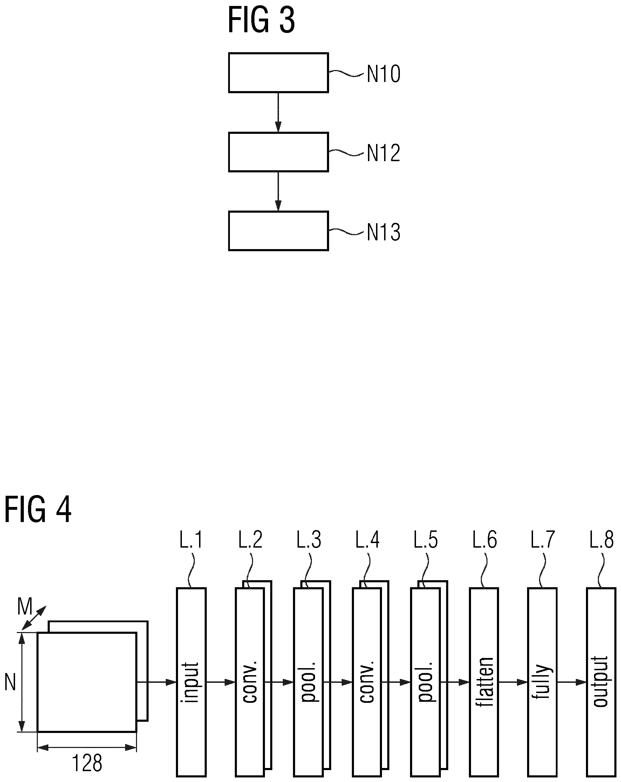

Die Figur 3 skizziert Schritte eines computerimplementierten Verfahrens zum Training eines maschinellen Navigatorsignal-Bewertung-Lernmodells, wobei das trainierte Navigatorsignal-Bewertung-Lernmodell eingerichtet ist, Navigatorsignale beispielsweise mit einem Qualitäts-Wert, zu bewerten. Das Training des maschinellen Navigatorsignal-Bewertung-Lernmodells kann beispielsweise mit einem überwachten Lernen erfolgen. In einem Schritt N10 wird eine Vielzahl von Teilmengen von Navigatorsignalen bereitgestellt. In einem weiteren Schritt N12 wird eine Vielzahl von Bewertungen zu den jeweiligen Teilmengen der Navigatorsignale bereitgestellt. In einem weiteren Schritt N13, bzw. in einer Abfolge von weiteren Schritten, wird das maschinelle Navigatorsignal-Bewertungs-Lernmodell mittels der Vielzahl der Teilmengen der Navigatorsignale als Eingangsgröße und der jeweils zugeordneten Qualitäts-Werte als Zielgröße, bzw. Ausgangsgröße, adaptiert, um eine Abweichung der Bewertung des jeweiligen Navigatorsignals von dem jeweiligen Qualitäts-Wert zu minimieren. Das Adaptieren des maschinellen Navigatorsignale-Bewertung-Lernmodells kann auf der Grundlage eines Backpropagation-Algorithmus erfolgen. Um eine Überanpassung zu verhindern, können Regularisierungsmethoden verwendet werden.

Die Figur 4 skizziert eine Architektur maschinellen Navigatorsignal-Bewertung-Lernmodells, welches in einer Ausführungsform als neuronales Faltungs-Netzwerk zur Bewertung von Navigatorsignalen ausgebildet sein kann, und eingerichtet ist, Navigatorsignale, beispielsweise mit einem Qualität-Wert, zu bewerten.

In der dargestellten Ausführungsform umfasst das neuronale Faltungs-Netzwerk eine Eingangsknoten-Schicht L1, eine erste Faltungsschicht L2 mit einer nachfolgenden Pooling-Schicht L3, eine zweite Faltungsschicht L4 mit einer nachfolgenden Pooling-Schicht L5, eine Flatten-Schicht L6, eine vollständig verbundene Schicht L7 und eine Ausgangsknoten-Schicht L8.

Die Eingangsschicht L1 kann eine variable Größe aufweisen. Beispielsweise kann die Eingangsschicht für eine Anzahl N von Navigatorsignalen einer Schicht, bzw. einem Abbildungsgebiet, mit jeweils einer beispielhaften Länge eines Navigatorsignals von 128 Pixel ausgebildet sein. Alternativ kann die Eingangsschicht für jeweils eine Anzahl N von Navigatorsignalen von jeweils einer Vielzahl von Schichten M, bzw. Abbildungsgebieten M, mit jeweils einer beispielhaften Länge von 128 Pixel ausgebildet sein, wenn die Navigatorsignale von benachbarten Abbildungsgebieten für die Vielzahl von Navigatorsignale gemeinsam betrachtet werden. Dann kann die Eingangsschicht L1 eine Anordnung in der Form (N, M,128) oder (N*M, 128) aufweisen. Insbesondere können die Navigatorsignale in der Eingangsschicht L1 so angeordnet werden, wie es den jeweiligen Aufnahmezeitpunkt oder der räumlichen Anordnung der jeweiligen Abbildungsgebiete entspricht. Dadurch können Korrelationen in Zeitrichtung, wie zum Beispiel kurze Bewegungen, die sich beispielsweise über Magnetresonanz-Echosignale von 8 von 20 Schichten innerhalb einer Repetitionszeit (TR-Periode) erstrecken können effektiv genutzt werden.

Die jeweilige Faltungsschicht zur Merkmalsextraktion kann als eine Verbindungsschicht zwischen einer anterioren Knotenschicht und einer posterioren Knotenschicht ausgestaltet sein. Die jeweilige Faltungsschicht ist insbesondere dadurch gekennzeichnet, dass die Struktur und die Gewichte der eingehenden Kanten eine Faltungsoperation auf der Basis einer bestimmten Anzahl von Kernen bilden. Der Kernel kann eine d-dimensionale Matrix, z. B. eine 2x2-Matrix sein, die klein ist im Vergleich zur Anzahl der Knoten der Faltungsschicht. Die jeweilige Faltungsschicht kann eine Vielzahl von Kanälen oder Feature Maps, wie z. B. 32 oder 64 Kanäle, aufweisen, insbesondere wenn eine Vielzahl von Kerneln in der Faltungsschicht verwendet werden. Eine Aktivierungsfunktion der jeweiligen Faltungsschicht kann in dieser Ausführungsform eine ReLU-Aktivierungsfunktion (ReLU: Akronym für "Rectified Linear Units") sein, andere Aktivierungsfunktionen wie z. B. ELU (Akronym für "Exponential Linear Unit"), LeakyReLU, Sigmoid, Tanh oder Softmax können alternativ verwendet werden.

Die jeweilige Pooling-Schicht L3 bzw. L5 kann als eine Verbindungsschicht zwischen einer anterioren Knotenschicht und einer posterioren Knotenschicht aufgefasst werden, die mittels einer nichtlinearen Pooling-Funktion benachbarter Knoten, wie zum Beispiel einer Max-Funktion, einer Mittelwert-Funktion oder einer L2-Norm, die Anzahl der Knoten reduziert. Das Pooling wird auf jede d-dimensionale Matrix der vorherigen Schicht angewandt. Die Pooling-Schicht erhält als Eingabe die Feature Maps, die als Ausgabe der Faltungsschichten gebildet werden und reduziert die Größe der Bilder, bzw. Vektoren, wobei gleichzeitig ihre wesentlichsten Merkmale erhalten bleiben. Mittels der Flatten-Schicht L6 können die Daten eindimensional angeordnet werden.

Die vollständig verbundene Schicht L7 kann als eine Verbindungsschicht zwischen einer vorderen Knotenschicht und einer hinteren Knotenschicht aufgefasst werden. Die vollständig verbundene Schicht kann dadurch charakterisiert werden, dass eine Mehrheit, insbesondere alle Kanten zwischen Knoten einer vorangehenden Knotenschicht und den Knoten einer nachfolgenden Knotenschicht vorhanden sind, und wobei das Gewicht jeder dieser Kanten individuell angepasst werden kann.

Mittels beispielsweise einer Sigmoid-Aktivierung, können die bewerteten Navigatorsignale, die entsprechend den zugeordneten Magnetresonanz-Echosignalen in der Ausgangsschicht L8 angeordnet sind, einen Wert zwischen 0 und 1 liefern. Das Navigatorsignal-Bewertungs-Lernmodell gibt dann für jede der N Magnetresonanz-Echosignale einen Wert zwischen 0 und 1 zur Ähnlichkeit zu den anderen Navigatoren aus.

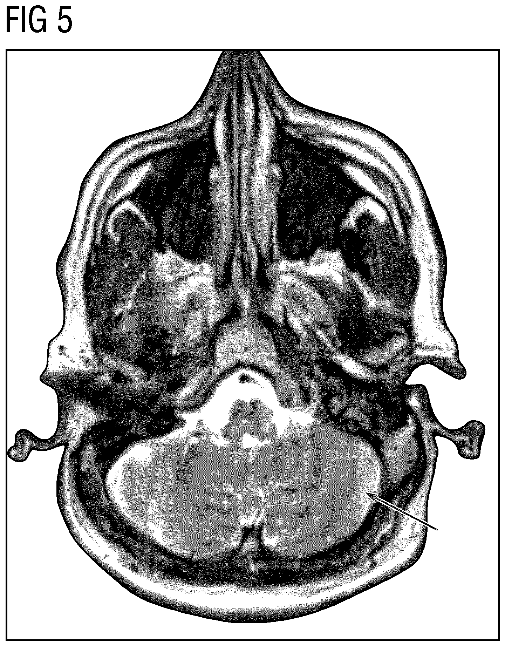

Die Figuren 5 bis 7 skizzieren drei Magnetresonanz-Bilder mit Bewegungsartefakten. Die Figur 5 repräsentiert ein Magnetresonanz-Bild, welches mit einer vollständigen Vielzahl von Magnetresonanz-Echosignal reproduziert wurde. Der Pfeil kennzeichnet einen Teilbereich des Magnetresonanz-Bildes, indem ein Bewegungsartefakten entsprechend einer schwarzen Struktur erkennbar ist.

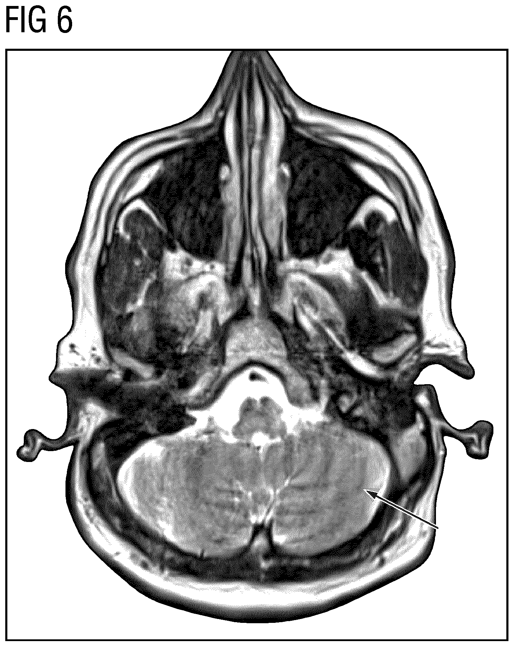

Die Figur 6 repräsentiert ein Magnetresonanz-Bild, welches mittels einer Korrelations-Bewertung der Magnetresonanz-Echosignale basierend auf eine Teilmenge der Vielzahl der Magnetresonanz-Echosignale der Figur 5 reproduziert wurde. Für die Bestimmung der Korrelations-Teilmenge wurden aus der Vielzahl von Magnetresonanz-Echosignalen zwei Magnetresonanz-Echosignale verworfen. Die zwei verworfenen Magnetresonanz-Echosignale wurden ausgewählt, indem eine Korrelation einer Vielzahl von Navigatorsignalen durchgeführt wurde, wobei die am wenigsten korrelierenden Navigatorsignale verworfen wurden. Die Vielzahl von Navigatorsignalen wurden dabei zusammen mit den jeweiligen Magnetresonanz-Echosignalen erfasst, um die Magnetresonanz-Echosignale zu identifizieren, die ein Bewegungsartefakten hervorrufen können. Wie in der Figur 6 zu erkennen ist, konnte damit das gekennzeichnete Bewegungsartefakten nicht aus dem Magnetresonanz-Bild entfernt werden.

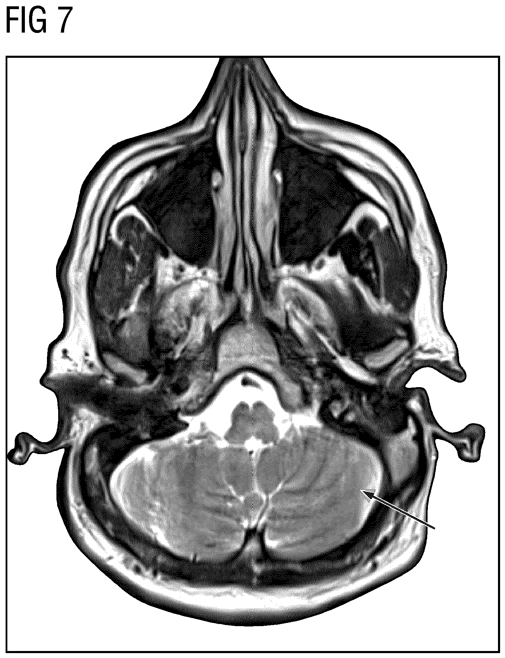

Die Figur 7 repräsentiert ein Magnetresonanz-Bild, bei dem durch Selektion von Navigatorsignalen eine Optimierungs-Teilmenge der Vielzahl der Magnetresonanz-Echosignal bestimmt werden konnte. Die Optimierungs-Teilmenge weist nur die Magnetresonanz-Echosignale auf, die der optimierten Auswahl von Navigatorsignale entsprechen. In der Reproduktion des Magnetresonanz-Bildes, die auf der Optimierung-Teilmenge der Vielzahl der Magnetresonanz-Echosignalen basiert, lassen sich in dem gekennzeichneten Teilbereich keine Bewegungsartefakte erkennen, womit das Magnetresonanz-Bild optimiert wurde. Somit kann gezeigt werden, dass durch eine geeignete Auswahl von Magnetresonanz-Echosignalen Bewegungsartefakte in den Magnetresonanz-Bildern reduziert werden können.

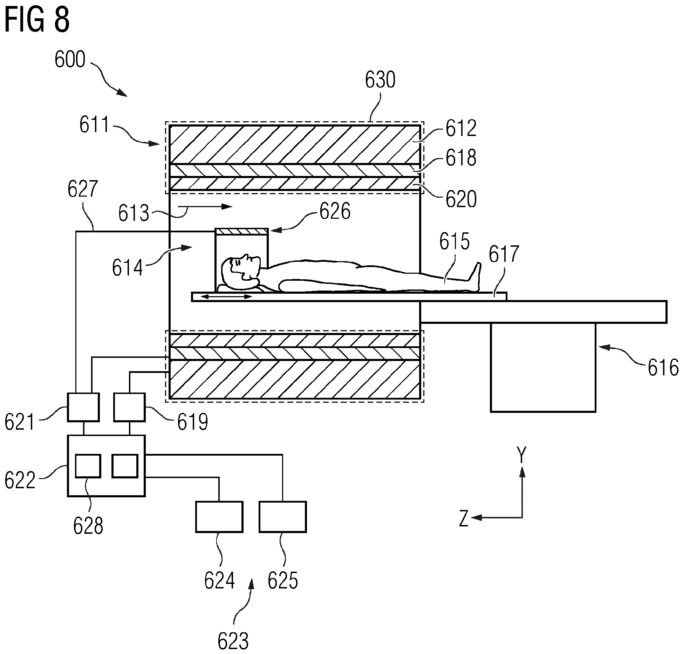

In Figur 8 ist eine Magnetresonanzanlage 600 dargestellt. Die Magnetresonanzanlage 600 umfasst eine Felderzeugungseinheit 611, welche einen Hauptmagneten 612 mit einem oder mehreren Permanentmagneten, Elektromagneten oder supraleitenden Magneten zur Erzeugung eines starken und insbesondere homogenen Hauptmagnetfelds 13 (B0-Magnetfeld) aufweist. Zudem umfasst die Magnetresonanzanlage 600 einen Patientenaufnahmebereich 614 zur Aufnahme eines Abbildungsgebietes eines Patienten 615. Der Patientenaufnahmebereich 614 ist in dem gezeigten Ausführungsbeispiel zylinderförmig ausgebildet und in einer Umfangsrichtung von dem Hauptmagneten 612 umschlossen. Grundsätzlich sind jedoch auch von diesem Beispiel abweichende Ausbildungen des Patientenaufnahmebereichs 614 vorstellbar. Der Patientenaufnahmebereich 614 kann im Wesentlichen mit einem Magnetresonanz-Bildaufnahmebereich der Magnetresonanzanlage 600 übereinstimmen.

In dem in Figur 8 gezeigten Beispiel ist der Patient 615 mittels einer Patientenlagerungsvorrichtung 616 der Magnetresonanzanlage 600 in dem Patientenaufnahmebereich 614 positionierbar. Die Patientenlagerungsvorrichtung 616 weist hierfür einen horizontal bewegbaren Patiententisch 617 auf.

Die Felderzeugungseinheit 611 weist ein Gradientensystem mit wenigstens einer Gradientenspule 618 zum Erzeugen eines magnetischen Gradientenfelds auf, welches für eine Ortskodierung während einer Magnetresonanzuntersuchung verwendet wird. Die Gradientenspule 618 wird mittels einer Gradientensteuereinheit 619 der Magnetresonanzanlage 600 angesteuert. Das Gradientensystem kann mehrere Gradientenspulen 618 zur Erzeugung von magnetischen Gradientenfeldern entlang unterschiedlicher, vorzugsweise orthogonal zueinander ausgerichteter, Raumrichtungen umfassen.

Die Felderzeugungseinheit 611 umfasst außerdem ein Hochfrequenzsystem mit einer Hochfrequenzspule, welche im vorliegenden Ausführungsbeispiel als fest in das Magnetresonanzgerät 600 integrierte Körperspule 620 ausgebildet ist. Die Körperspule 620 ist zu einer Anregung von Kernspins ausgelegt, die sich in dem von dem Hauptmagneten 612 erzeugten Hauptmagnetfeld 613 befinden. Die Körperspule 620 wird von einer Hochfrequenzsteuereinheit 621 des Magnetresonanzgeräts 600 angesteuert und kann hochfrequente Anregungspulse in den Bildaufnahmebereich einstrahlen, der im Wesentlichen von dem Patientenaufnahmebereich 614 des Magnetresonanzgeräts 600 gebildet ist, ein. Die Körperspule 620 kann weiterhin zum Empfang von Magnetresonanzsignalen ausgebildet sein und eine Empfangseinheit oder einen Teil einer Empfangseinheit des Magnetresonanzgeräts 600 bilden.

Zu einer Steuerung des Magnetresonanzgeräts 600, insbesondere der Gradientensteuereinheit 619 und der Hochfrequenzsteuereinheit 621, weist das Magnetresonanzgerät 600 eine Steuereinheit 622 auf. Die Steuereinheit 622 ist insbesondere dazu ausgebildet, eine Durchführung einer Bildgebungssequenz, wie z. B. einer GRE (gradient echo) Sequenz, einer TSE (turbo spin echo) Sequenz oder einer UTE (ultra-short echo time) Sequenz, zu koordinieren. Zudem umfasst die Steuereinheit 622 eine Recheneinheit 628 zu einer Auswertung von Magnetresonanzsignalen, die während einer Magnetresonanzuntersuchung mit einer Bildgebungssequenz erfasst werden.

Das Magnetresonanzgerät 600 kann eine Benutzerschnittstelle 623 umfassen, welche eine Signalverbindung mit der Steuereinheit 622 aufweist. Steuerinformationen, wie z. B. Bildgebungsparameter der Magnetresonanzuntersuchung, können auf einer Anzeigeeinheit 624, beispielsweise auf zumindest einem Monitor, der Benutzerschnittstelle 623 angezeigt werden. Die Anzeigeeinheit 624 kann insbesondere dazu ausgelegt sein, eine grafische Benutzeroberfläche mit der Darstellung einer relevanten Körperregion des Patienten 615 bereitzustellen. Weiterhin weist die Benutzerschnittstelle 623 eine Eingabeeinheit 625 auf, mittels welcher Parameter einer Magnetresonanzmessung von einem Nutzer eingegeben oder verändert werden können.

Das Magnetresonanzgerät 600 kann weitere Komponenten, wie z. B. eine Lokalspule 626, aufweisen. Die Lokalspule 626 kann in einer anwendungsgemäßen Position an einer diagnostisch oder therapeutisch relevanten Körperregion des Patienten 615 positioniert sein. Die Lokalspule 626 weist vorzugsweise eine Mehrzahl von Antennenelementen auf, welche dazu ausgebildet sind, Magnetresonanzsignale der relevanten Körperregion des Patienten 615 zu erfassen und an die Recheneinheit 628 und/oder die Steuereinheit 622 zu übermitteln. Die Lokalspule kann hierfür mittels einer elektrischen Anschlussleitung 627 oder einer anderen Signalverbindung mit der Hochfrequenzsteuereinheit 621 und der Steuereinheit 622 verbunden sein. Analog zu der Körperspule 620 kann auch die Lokalspule 626 zu einem Anregen von Kernspins in einer Körperregion 631 des Patienten 615 ausgebildet sein. Die Lokalspule 626 kann hierfür von der Hochfrequenzsteuereinheit 621 angesteuert werden.

Üblicherweise sind die Felderzeugungseinheit 611 sowie eine Magnethaltestruktur von einem Gehäuse 630 umschlossen. Das Gehäuse 630 kann dazu ausgebildet sein, Komponenten des Magnetresonanzgeräts 600 vor äußeren Einflüssen zu schützen und/oder einen Berührschutz für einen Patienten 615 bereitzustellen.

Die vorliegende Erfindung ist nicht auf die vorhergehend beschriebene Ausführungsform beschränkt, solange sie vom Gegenstand der nachfolgenden Ansprüche umfasst ist. Ergänzend wird darauf hingewiesen, dass die Begriffe "umfassend" und "aufweisend" keine anderen Elemente oder Schritte ausschließen und die unbestimmten Artikel "eine" oder "ein" keine Vielzahl ausschließen. Ferner wird darauf hingewiesen, dass Merkmale oder Schritte, die mit Verweis auf obige Ausführungsformen beschrieben worden sind, auch in Kombination mit anderen Merkmalen verwendet werden können.

Darüber hinaus wird darauf hingewiesen, dass unabhängig vom grammatikalischen Geschlecht eines bestimmten Begriffs, Personen mit männlicher, weiblicher oder anderer Geschlechteridentität mit umfasst sind.

## Patentansprüche

1. Computerimplementiertes Verfahren zur Erstellung eines optimierten Magnetresonanz-Bildes aufweisend:
Bereitstellen einer Vielzahl von Magnetresonanz-Echosignalen eines Magnetresonanz-Bildgebungsvorgangs eines Abbildungsgebietes (S10);
Bereitstellen einer Vielzahl von Navigatorsignalen, die zusammen mit den jeweiligen Magnetresonanz-Echosignalen erfasst wurden, und jeweils den jeweiligen Magnetresonanz-Echosignalen zugeordnet sind (S12);
Bestimmen eines Qualität-Werts des jeweiligen Navigatorsignals der Vielzahl der Navigatorsignale mittels eines trainierten maschinellen Navigatorsignal-Bewertungs-Lernmodells, insbesondere zur Bewertung des jeweiligen Navigatorsignals (S14);
Bestimmen einer Teilmenge der Vielzahl der Magnetresonanz-Echosignale, basierend auf den jeweiligen Qualitäts-Werten der Navigatorsignale, um insbesondere das Magnetresonanz-Bild zu verbessern (S16); und
Generieren des optimierten Magnetresonanz-Bildes mittels der Teilmenge der Vielzahl von Magnetresonanz-Echosignalen (S18).

2. Verfahren gemäß Anspruch 1, wobei die Teilmenge der Vielzahl der Magnetresonanz-Echosignale mittels Verwerfens zumindest eines Magnetresonanz-Echosignals basierend auf dem jeweiligen Qualitäts-Wert aus der Vielzahl der Magnetresonanz-Echosignale bestimmt wird, wobei insbesondere eine maximale Anzahl von verworfenen Magnetresonanz-Echosignalen begrenzt ist.

3. Verfahren gemäß Anspruch 2, wobei eine Anzahl verworfener Magnetresonanz-Echosignale, die in einem k-Raum benachbart angeordnet sind, begrenzt ist; und insbesondere nicht mehr in dem k-Raum benachbart Magnetresonanz-Echosignale verworfen werden als einer Vervielfachung um einen festgelegten Faktor einer lokalen Unterabtastung der Magnetresonanz-Echosignale in dem k-Raum entspricht.

4. Verfahren gemäß einem der Ansprüche 2 oder 3, wobei zumindest ein Magnetresonanz-Echosignal mit einem Qualitäts-Wert, der kleiner als ein Limit-Wert ist, verworfen wird.

5. Verfahren gemäß einem der vorangehenden Ansprüche, wobei das Navigatorsignal mittels eines Kamerasignals; und/oder eines Videosignals; und/oder eines Radarsignals; und/oder eines RF-Reflexions-Signals; und/oder eines Pilot-Tone-Signals; und/oder eines Navigatorsignals, welches auf einem Magnetresonanz-Signal basiert, generiert wird.

6. Computerimplementiertes Verfahren zur Bewertung eines Magnetresonanz-Echosignals, insbesondere in Bezug auf eine Qualität eines Magnetresonanz-Bildes, aufweisend:
Bereitstellen einer Vielzahl von Magnetresonanz-Echosignalen eines Magnetresonanz-Bildgebungsvorgangs eines Abbildungsgebietes (S20);
Bereitstellen einer Vielzahl von Navigatorsignalen, die jeweils mit den Magnetresonanz-Echosignalen der Vielzahl der Magnetresonanz-Echosignalen assoziiert sind (S21);
Erstellen einer Vielzahl von ersten Teilmengen der Vielzahl der Magnetresonanz-Echosignale, wobei aus jeder ersten Teilmenge zumindest ein Magnetresonanz-Echosignal der Vielzahl der Magnetresonanz-Echosignale verworfen ist; und jede der Vielzahl der ersten Teilmengen der Magnetresonanz-Echosignale in Bezug auf die enthaltenen Magnetresonanz-Echosignale unterschiedlich ist (S22);
Rekonstruieren einer Vielzahl von ersten Magnetresonanz-Bildern, wobei jedes erste Magnetresonanz-Bild der Vielzahl der ersten Magnetresonanz-Bilder auf einer anderen ersten Teilmenge der Vielzahl der Magnetresonanz-Echosignale basiert (S23);
Bestimmen eines Qualitäts-Werts jedes ersten Magnetresonanz-Bildes der Vielzahl der ersten Magnetresonanz-Bilder (S24), wobei insbesondere der Qualität-Wert jedes ersten Magnetresonanz-Bildes mittels eines Bildqualität-Bewertungs-Netzwerks bestimmt wird; und
Zuweisen des Qualitäts-Werts des jeweiligen ersten Magnetresonanz-Bildes zu der jeweiligen ersten Teilmenge der assoziierten Navigatorsignale (S25); besonders vorzugsweise wenn der Qualitäts-Wert zumindest eines ersten Magnetresonanz-Bildes größer ist als ein Qualität-Wert eines originären Magnetresonanz-Bildes, welches mittels der Vielzahl der bereitgestellten Magnetresonanz-Echosignale des Bildgebungsvorgangs rekonstruiert ist; um insbesondere bewertete Navigatorsignale bereitzustellen (S26).

7. Verfahren gemäß Anspruch 6, aufweisend:
Zuweisen eines um den Qualitäts-Wert des jeweiligen ersten Magnetresonanz-Bildes verminderten maximalen Qualitäts-Wert zu dem jeweiligen assoziierten Navigatorsignal des jeweiligen zumindest einen verworfenen Magnetresonanz-Echosignal, um insbesondere bewertete Navigatorsignale bereitzustellen.

8. Verfahren gemäß Anspruch 6 oder 7, wobei die Vielzahl von Magnetresonanz-Echosignale, Magnetresonanz-Echosignale eines Magnetresonanz-Bildgebungsvorgangs zumindest eines dem Abbildungsgebiet benachbarten Abbildungsgebietes umfassen; und/oder wobei die Vielzahl von Magnetresonanz-Echosignale Magnetresonanz-Echosignale einer Vielzahl von zeitlich aufeinanderfolgenden Magnetresonanz-Bildgebungsvorgängen des Abbildungsgebiets umfasst.

9. Verfahren gemäß einem der Ansprüche 6 bis 8, aufweisend:
Rekonstruieren eines originären Magnetresonanz-Bildes mittels der Vielzahl der bereitgestellten Magnetresonanz-Echosignale des Bildgebungsvorgangs eines Abbildungsgebietes (S30);
Bestimmen eines Qualitäts-Werts des originären Magnetresonanz-Bildes, insbesondere mittels des Bildqualität-Bewertungs-Netzwerks (S31);
Vergleichen des Qualitäts-Werts jedes der ersten Magnetresonanz-Bilder mit dem Qualitäts-Wert des originären Magnetresonanz-Bildes, basierend auf einer Höhe des jeweiligen Qualitäts-Wertes (S32); und, wenn der Vergleich das originären Magnetresonanz-Bild präferiert (S33), weiterhin aufweisend:
Erstellen einer Vielzahl von zweiten Teilmengen der Vielzahl der Magnetresonanz-Echosignale, mittels Verwerfen zumindest eines weiteren Magnetresonanz-Echosignals aus jeder ersten Teilmenge der Vielzahl der Magnetresonanz-Echosignale; und wobei jede zweite Teilmenge der Vielzahl der Magnetresonanz-Echosignale in Bezug auf die enthaltenen Magnetresonanz-Echosignale unterschiedlich ist (S34);
Rekonstruieren einer Vielzahl von zweiten Magnetresonanz-Bildern, wobei jedes zweite Magnetresonanz-Bild der Vielzahl der zweiten Magnetresonanz-Bilder auf einer anderen zweiten Teilmenge der Vielzahl der Magnetresonanz-Echosignale basiert (S35);
Bestimmen eines Qualität-Werts jedes der Vielzahl der zweiten Magnetresonanz-Bilder mittels des Bildqualität-Bewertungs-Netzwerks (S36); und
Zuweisen des Qualitäts-Werts des jeweiligen zweiten Magnetresonanz-Bildes der Vielzahl der zweiten Magnetresonanz-Bilder zu einer jeweiligen zweiten Teilmenge der assoziierten Navigatorsignale (S37), besonders vorzugsweise wenn der Qualitäts-Wert zumindest eines zweiten Magnetresonanz-Bildes größer ist als ein größter Qualitäts-Wert zumindest eines ersten Magnetresonanz-Bildes, um insbesondere eine Vielzahl von bewerteten Navigatorsignalen bereitzustellen (S38).

10. Verfahren gemäß einem der Ansprüche 7 bis 9, aufweisend:
Auswahl eines Referenz-Magnetresonanz-Bildes aus der Vielzahl der zweiten Magnetresonanz-Bilder, basierend auf einer Höhe des jeweiligen Qualitäts-Wertes (S40);
Vergleichen des Referenz-Magnetresonanz-Bildes mit zumindest einer Teilmenge der ersten Magnetresonanz-Bilder, basierend auf einer Höhe des jeweiligen Qualitäts-Wertes (S41); und, wenn der Vergleich das Referenz-Magnetresonanz-Bildes präferiert (S42), weiterhin aufweisend:
Erstellen einer Vielzahl von dritten Teilmengen der Vielzahl der Magnetresonanz-Echosignale, mittels Verwerfen zumindest eines weiteren Magnetresonanz-Echosignals aus jeder zweiten Teilmenge der Vielzahl der Magnetresonanz-Echosignale; und wobei jede dritte Teilmenge der Vielzahl der Magnetresonanz-Echosignale in Bezug auf die enthaltenen Magnetresonanz-Echosignale unterschiedlich ist (S43);
Rekonstruieren einer Vielzahl von dritten Magnetresonanz-Bildern, wobei jedes dritte Magnetresonanz-Bild der Vielzahl der dritten Magnetresonanz-Bilder auf einer anderen dritten Teilmenge der Vielzahl der Magnetresonanz-Echosignale basiert (S44);
Bestimmen eines Qualität-Werts jedes der Vielzahl der dritten Magnetresonanz-Bilder, insbesondere mittels des Bildqualität-Bewertungs-Netzwerks (S45); und
Zuweisen der Bewertung des jeweiligen dritten Magnetresonanz-Bildes der Vielzahl der dritten Magnetresonanz-Bilder zu jeweiligen dritten Teilmengen der assoziierten Navigatorsignale (S46), um insbesondere eine Vielzahl von bewerteten Navigatorsignalen bereitzustellen (S47).

11. Computerimplementiertes Verfahren zum Trainieren eines maschinellen Navigatorsignal-Bewertungs-Lernmodells zur Bewertung eines Navigatorsignals mit einem Qualitäts-Wert, aufweisend:
Bereitstellen einer Vielzahl von Teilmengen von Navigatorsignalen (N10);
Bereitstellen einer Vielzahl von Bewertungen zu den jeweiligen Teilmengen der Navigatorsignale (N12);
Adaptieren des maschinellen Navigatorsignal-Bewertungs-Lernmodells mittels der Vielzahl der Teilmengen der Navigatorsignale als Eingangsgröße und dem jeweils zugeordneten Qualitäts-Wert als Zielgröße/Ausgangsgröße, um eine Abweichung der Bewertung des jeweiligen Navigatorsignals von dem jeweiligen Qualitäts-Wert zu minimieren (N13).

12. Magnetresonanzanlage (600) umfassend,
einen Hauptmagneten (612);
ein Gradientensystem;
ein Hochfrequenzsystem; und
eine Steuereinheit (622), wobei die Steuereinheit (622) mit dem Hauptmagneten, dem Gradientensystem und dem Hochfrequenzsystem gekoppelt ist, und wobei die Steuereinheit (622) eingerichtet ist, ein Verfahren nach einem der Ansprüche 1 bis 11 mittels der Magnetresonanzanlage (600) auszuführen.

13. Computerprogramm, welches direkt in einen Speicher einer Steuereinheit (622) einer Magnetresonanzanlage (600) ladbar ist, mit Programm-Mitteln, um die Schritte eines der Verfahren gemäß Ansprüchen 1 bis 11 auszuführen, wenn das Programm in der Steuereinheit (622) der Magnetresonanzanlage (600) ausgeführt wird.

14. Elektronisch lesbarer Datenträger mit darauf gespeicherten elektronisch lesbaren Steuerinformationen, welche zumindest ein Computerprogramm nach Anspruch 13 umfassen und derart ausgestaltet sind, dass sie bei Verwendung des Datenträgers in einer Steuereinheit (622) einer Magnetresonanzanlage (600) ein Verfahren gemäß einem der Ansprüche 1 bis 11 durchführen.

15. Verwendung eines Navigatorsignals, welches zusammen mit einem Magnetresonanz-Echosignal erfasst wurde, zur Bestimmung einer Teilmenge von Magnetresonanz-Echosignalen aus einer Vielzahl von Magnetresonanz-Echosignalen für eine Rekonstruktion eines verbesserten Magnetresonanz-Bildes gemäß einem der Ansprüche 1 bis 5.

16. Computerimplementiertes Verfahren zur Erstellung eines optimierten Magnetresonanz-Bildes gemäß einem der Ansprüche 1 bis 5;
wobei das maschinelle Navigatorsignal-Bewertungs-Lernmodell, mittels eines computerimplementierten Verfahrens, gemäß Anspruch 11, mit bewerteten Navigatorsignalen trainiert wird; und
wobei die Navigatorsignale mittels eines computerimplementierten Verfahrens zur Bewertung eines Magnetresonanz-Echosignals, gemäß einem der Ansprüche 6 bis 10, bewertet werden, um bewertete Navigatorsignale bereitzustellen.
